(19) Europäisches Patentamt
European Patent Office
Office européen des brevets

(11) Veröffentlichungsnummer : **0 472 071 B1**

(12) # EUROPÄISCHE PATENTSCHRIFT

(45) Veröffentlichungstag der Patentschrift : **30.06.93 Patentblatt 93/26**

(51) Int. Cl.$^5$ : **B01J 31/18,** C07C 45/50

(21) Anmeldenummer : **91113446.8**

(22) Anmeldetag : **10.08.91**

(54) **Rhodiumhydroformylierungskatalysatoren mit Bis-phosphit-Liganden.**

(30) Priorität : **21.08.90 DE 4026406**

(43) Veröffentlichungstag der Anmeldung : **26.02.92 Patentblatt 92/09**

(45) Bekanntmachung des Hinweises auf die Patenterteilung : **30.06.93 Patentblatt 93/26**

(84) Benannte Vertragsstaaten : **BE CH DE ES FR GB IT LI NL SE**

(56) Entgegenhaltungen :
**EP-A- 0 213 639**
**EP-A- 0 214 622**
**EP-A- 0 353 770**
**FR-A- 2 236 856**

(73) Patentinhaber : **BASF Aktiengesellschaft**
**Carl-Bosch-Strasse 38**
**W-6700 Ludwigshafen (DE)**

(72) Erfinder : **Lorz, Peter Michael, Dr.**
**L 4,10**
**W-6800 Mannheim 1 (DE)**
Erfinder : **Bertleff, Werner, Dr.**
**Franz-Marc-Strasse 12**
**W-6806 Viernheim (DE)**
Erfinder : **Roeper, Michael, Dr.**
**Albert-Schweitzer-Strasse 10**
**W-6706 Wachenheim (DE)**
Erfinder : **Koeffer, Dieter, Dr.**
**Zinkgraefstrasse 1**
**W-6940 Weinheim (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Rhodiumkatalysatoren mit speziellen Bisphosphiten als Liganden und deren Verwendung bei der Hydroformylierung.

Unter Carbonylierungsreaktionen versteht man die Produktion von sauerstoffhaltigen Produkten durch Umsetzung einer organischen Verbindung mit Kohlenmonoxid und vorzugsweise einem weiteren Reaktionspartner - vor allem Wasserstoff - in Anwesenheit eines Katalysators. Eine technisch besonders wichtige Reaktion ist die Hydroformylierung von Olefinen durch Umsetzung mit Kohlenmonoxid und Wasserstoff unter Bildung von Aldehyden, die ein Kohlenstoffatom mehr als die Edukte enthalten. Als Katalysatoren verwendet man Gruppe-VIII-Übergangsmetallkomplexe, die phosphorhaltige Liganden, z.B. Phosphine enthalten (siehe J. Falbe, New Syntheses with Carbon Monoxide, Springer Verlag, New York 1980).

Neben Kobaltkatalysatoren haben in den letzten Jahren zunehmend Rhodium-Katalysatoren zur Hydroformylierung niederer alpha-Olefine Bedeutung erlangt, da sie eine Reaktionsführung bei niedrigeren Drücken zulassen. Als Phosphorligand wird in der Regel Triphenylphosphin im Überschuß verwendet, wobei ein hohes Ligand/Rhodium-Verhältnis erforderlich ist, um die Selektivität der Reaktion zum kommerziell erwünschten n-Aldehydprodukt zu erhöhen.

In den letzten Jahren wurde der Versuch unternommen, wirksamere Phosphorliganden für die Hydroformylierung zu finden. Neben unterschiedlich substituierten Phosphinen wurden auch Phosphite auf ihre Eignung als Katalysatoren untersucht. Phosphite ergeben bei ihrer Koordination an ein Übergangsmetallzentrum Katalysatoren von gesteigerter Aktivität, doch ist das Standzeitverhalten dieser Katalysatorsysteme wegen der starken Hydrolyseempfindlichkeit der Phosphitliganden unbefriedigend. Eine drastisch verringerte Hydrolyseempfindlichkeit sollen durch Bisaryldiole substituierte chelatisierende Polyphosphite aufweisen, wie sie in EP-A-214622 beschrieben werden. Die Rhodiumkomplexe dieser Liganden sollen äußerst aktive Hydroformylierungskatalysatoren bilden. EP-A-213639 beschreibt Chelat-Bisphosphite, die an einem Phosphoratom Diorgano-und am zweiten Phosphoratom Triorganophosphitfunktionalität aufweisen. Aus EP-A-155508 ist ferner die Verwendung von bisaryldiolsubstituierten Monophosphiten bei der rhodiumkatalysierten Hydroformylierung von sterisch gehinderten Olefinen, z. B. Isobuten bekannt.

Diol- und triolsubstituierte Monophosphite und ihr Einsatz bei der Hydroformylierung werden schließlich in EP-A2-149894, EP-A2-096988 und EP-A2-096986, beschrieben.

Obgleich die genannten Bis-phosphite sehr gute Komplexliganden für Rhodiumhydroformylierungs-Katalysatoren sind, bestand die Aufgabe, deren Wirksamkeit und Hydrolysebeständigkeit noch weiter zu verbessern.

Diese Aufgabe wurde mit verbesserten Rhodiumhydroformylierungskatalysatoren mit Bis-phosphit-liganden der Formel I

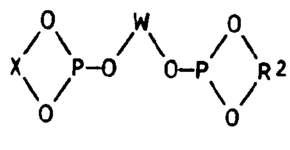

I,

gelöst, in der

| | |
|---|---|
| -X- | einen zweiwertigen Bisarylenrest oder $R^1$, |
| W | einen zweiwertigen Arylen-, Bisarylen- oder Alkylen-Rest und |
| $R^1$ und $R^2$ | gleich oder verschieden sind und einen substituierten oder unsubstituierten Alkylen oder ortho-Arylenrest bedeuten. |

Von den Verbindungen der Formel 1 sind solche bevorzugt, bei denen die Reste X und W in der Formel I Bis-arylenreste, insbesondere den Rest der Formel II

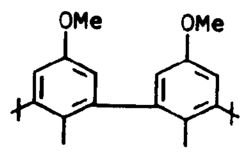

II

und $R^2$ einen o-Phenylen-, 2,2-Dimethyl-propylen-1,3- oder 1,1,2,2-Tetramethyl-ethylen-Rest bedeuten. Ferner sind solche Verbindungen der Formel I hervorzuheben, bei denen W, $R^1$ und $R^2$ unabhängig voneinander

o-Phenylen-, 2,2-Dimethyl-propylen-1,3- oder 1,1,2,2-Tetramethyl-ethylen-Reste bedeuten.

Symmetrische Verbindungen, d.s. solche bei denen X und $R^2$ identisch sind, sind bevorzugt, da sie sich besonders leicht herstellen lassen.

Die erfindungsgemäß zu verwendenden Bis-phosphite können nach an sich bekannten Methoden durch eine geeignet gewählte Abfolge von Phosphorhalogenid-Alkoholkondensationsreaktionen hergestellt werden.

a) Beispielsweise setzt man Phosphortrichlorid mit einem Diol unter Bildung eines Monochlorphosphites um;

b) setzt dieses Zwischenprodukt mit einem weiteren Diol unter Bildung des korrespondierenden hydroxylsubstituierten Diorganophosphites um;

c) läßt dieses Diorganophosphitzwischenprodukt mit Phosphortrichlorid unter Bildung des korrespondierenden Phosphordichloridzwischenproduktes reagieren;

d) und schließt mit der Reaktion dieses Dichlorids mit einem entsprechenden Diol unter Bildung des gewünschten Bisphosphites ab.

Während dieser Syntheseweg für die Herstellung von unsymmetrisch substituierten Phosphiten erforderlich ist, lassen sich symmetrisch substituierte Verbindungen durch Reaktion des Produktes von Schritt a) mit einem entsprechenden Diol im Molverhältnis 2:1 herstellen.

Die genannten Kondensationsreaktionen werden in der Regel in einem geeigneten Lösungsmittel, z.B. Toluol in Gegenwart einer Hilfsbase, z.B. Triethylamin, als HCl-Akzeptor durchgeführt.

Die so hergestellten Bisphosphitliganden sind hervorragend geeignet für die Herstellung von Rhodiumkomplexen für Verfahren zur Carbonylierung, besonders für die Hydroformylierung. Sie zeichnen sich durch hohe Hydrolysestabilität und vor allem in Verbindung mit Rhodium als Katalysatormetall durch hohe katalytische Aktivität aus. Aufgrund ihres hohen Molekulargewichtes besitzen sie eine geringe Flüchtigkeit und lassen sich deshalb gut von den leichtflüchtigen Reaktionsprodukten abtrennen. Sie sind in allen gängigen organischen Solventien gut löslich. Sie sind insbesondere geeignet zur Hydroformylierung von alpha- und internen Olefinen einschließlich Isobuten und es lassen sich je nach eingesetztem Phosphit Aldehyde mit einem weiten Bereich der Selektivität von niedrigen bis hohen Anteilen an n-Produkt einstellen.

Die Bisphosphitliganden dieser Erfindung werden sowohl als Liganden des Gruppe-VIII-Übergangsmetallkomplexes als auch als freier Phosphorligand, der vorzugsweise im Reaktionsmedium vorhanden ist, eingesetzt. Es ist auch nicht erforderlich, daß der Phosphorligand des Gruppe-VIII-Übergangsmetallbisphosphitkomplexes und der überschüssige freie Ligand identisch sind, obgleich dies in der Regel der Fall ist. Ferner können auch Mischungen verschiedener Liganden als freie Ligandkomponente vorhanden sein. Der Hydroformylierungsprozeß kann mit jeder überschüssigen Menge an freien Bisphosphitliganden durchgeführt werden; das Vorhandensein von freiem Bisphosphitliganden ist allerdings nicht unbedingt erforderlich.

Im allgemeinen werden ungefähr 2 bis 100 und vorzugsweise 3 bis 50 Mol Ligand pro Mol Gruppe-VIII-Übergangsmetall, d.i. die Summe aus dem komplexierten und freien Liganden, eingesetzt. Frischer Ligand kann zu jedem Zeitprodukt der Reaktion zugesetzt werden, um die Konzentration an freiem Liganden konstant zu halten. Die Übergangsmetall-Bisphosphitkomplex-Katalysatoren können vor ihrem Einsatz synthetisiert werden. In der Regel werden aber die katalytisch aktiven Komplexe aus einem Katalysatorvorläufer wie $Rh(CO)_2$acetylacetonat, $Rh_2O_3$, $Rh_4(CO)_{12}$, $Rh_6(CO)_{16}$, $Rh(NO_3)_2$, $Rh(OAc)_2$ und dem Bisphosphitliganden im Reaktionsmedium gebildet. Vorzugsweise werden $Rh(CO)_2$acetylacetonat oder $Rh(OAc)_2$ als Rhodiumkomponente eingesetzt, die in Gegenwart eines Lösungsmittels mit dem Bisphosphitliganden umgesetzt wird, um den Vorläufer des katalytisch aktiven Komplexes zu bilden, der in den Reaktor zusammen mit überschüssigem Ligand eingebracht wird, um dort unter Reaktionsbedingungen in situ zum aktiven Katalysator abzureagieren.

Die Konzentration des Gruppe-VIII-Komplexkatalysators beträgt im allgemeinen zwischen 10 und 1000 ppm, berechnet als freies Metall, vorzugsweise 10 bis 500 ppm Rh und insbesondere 25 bis 350 ppm Rh. Die zu hydroformylierenden Olefine, können terminal oder intern ungesättigt sein, geradkettig, verzweigt oder von cyclischer Struktur sein. Sie können 2 bis 20 C-Atome enthalten und auch mehrere ethylenisch ungesättigte Gruppen aufweisen.

Die Carbonylierung und vorzugsweise Hydroformylierung wird im allgemeinen in einem organischen Lösungsmittel für den Gruppe-VIII-Übergangsmetallkomplex durchgeführt, das unter den Reaktionsbedingungen inert ist. Die Lösungsmittelmenge ist nicht kritisch und muß nur ausreichend sein, um die erforderliche Rhodiummenge im Reaktionsmedium zu lösen.

Die Hydroformylierung in Gegenwart der neuen Phosphite wird bei Temperaturen von 30 bis 200 °C und Drücken von 0,05 bis 700 bar durchgeführt.

Im allgemeinen führt der Einsatz der neuen Bisphosphite zu einer Absenkung der erforderlichen Reaktionstemperaturen. Bei der Hydroformylierung von Olefinen wird vorzugsweise ein Druck von weniger als 100 bar gewählt oder noch besser von weniger als 35 bar. Der Minimaldruck wird hauptsächlich durch die Gesamtmenge der Reaktanden bestimmt, die erforderlich ist, um die Reaktion aufrechtzuerhalten.

Im allgemeinen liegt das $H_2$:CO-Molverhältnis zwischen 1:10 bis 100:1 oder höher, vorzugsweise aber zwischen 1:1 und 40:1. Die Reaktionstemperaturen sollen 20 bis 200 °C betragen, im allgemeinen aber zwischen 30 und 130 °C liegen. Vorzugsweise erfolgt die Hydroformylierung von alpha-Olefinen zwischen 40 und 130 °C, während weniger reaktive Olefine wie Isobuten und interne Olefine oder Mischungen von alpha-Olefinen und internen Olefinen zwischen 50 und 130 °C hydroformyliert werden.

Eine zusätzliche Stabilisierung der erfindungsgemäß zu verwendenden Bisphosphite kann dadurch erfolgen, daß man dem Hydroformylierungsmedium ein Arylthiol der Formel ArSH, in der Ar eine substituierte oder unsubstituierte Arylgruppe ist, hinzufügt.

Die folgenden Beispiele sollen zur Erläuterung der vorliegenden Erfindung dienen.

Beispiel 1

Propen wurde in Gegenwart einer Mischung aus Rh(CO) $_2$acetylacetonat und dem Bisphosphitliganden der Formel (1) in 100 ml Texanol® in einem magnetisch gerührten 300 ml-Autoklaven hydroformyliert, wobei die Reaktionsbedingungen wie folgt waren: 90 ppm Rhodium, 70°C, 10 bar CO:$H_2$ (Molverhältnis 1:1), 30 bar Gesamtdruck, 4 h, 1,0 mol Propen. Die Bestimmung der Reaktionsprodukte erfolgte gaschromatographisch.

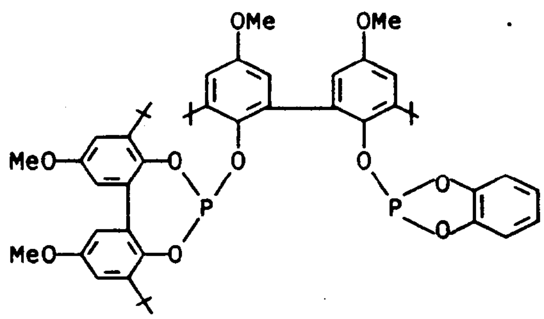

1

Man erhielt bei einem L/Rh-Verhältnis =5,0 (mol/mol) eine Ausbeute an Butyraldehyd von 96 % mit einem n-Anteil von 59 %.

Der Bisphosphitligand (1) wurde auf folgende Weise synthetisiert:

Aus einer Mischung von 10,0 g (28 mmol) 2,2'-Dihydroxy-3,3'-di-tert.-butyl-5,5'-dimethoxy-1,1'-biphenyl mit 400 ml Toluol wurden 70 ml Toluol zur azeotropen Abtrennung von Wasser abdestilliert; die erhaltene Lösung wurde mit 39 ml (280 mmol) Triethylamin versetzt und bei -20°C zu einer Lösung von 3,9 g (28 mmol) Phosphortrichlorid in 100 ml Toluol innerhalb 1 h zugetropft. Anschließend wurde die Mischung auf Raumtemperatur gebracht und nach einstündigem Rühren 2 h zum Rückfluß erhitzt. Zu dieser Reaktionsmischung wurden bei -20°C innerhalb 30 min 10,0 g (28 mmol) 2,2'-Dihydroxy-3,3'-di-tert.-butyl-5,5'-dimethoxy-1,1'-biphenyl und 39 ml (280 mmol) Triethylamin in 200 ml Toluol zugetropft. Die Mischung wurde langsam auf Raumtemperatur gebracht und 4 h zum Rückfluß erhitzt. Anschließend wurden 3,9 g (28 mmol) Phosphortrichlorid in 50 ml Toluol zugetropft. Nach 1 h Rühren bei Raumtemperatur wurde die Mischung 2 h zum Rückfluß erhitzt. Schließlich wurden bei -20°C 3,1 g (28 mmol) 1,2-Dihydroxybenzol und 39 ml (280 mmol) Triethylamin in 200 ml Toluol innerhalb von 30 min zugetropft. Nach einstündigem Rühren bei Raumtemperatur wurde 5 h zum Rückfluß erhitzt.

Die Reaktionsmischung wurde heiß abfiltriert und das klare Filtrat im Vakuum vom Lösungsmittel befreit. Der Rückstand wurde zweimal mit jeweils 50 ml Acetonitril extrahiert. Bei dem verbleibenden weißen Produkt handelte es sich um (1) (Schmelzpunkt: 207°C), das mit 58 % Ausbeute isoliert werden konnte. Der Strukturvorschlag für 1 konnte spektroskopisch und elementaranalytisch abgesichert werden.

$^{31}$P-NMR-Daten ($CDCl_3$): $\delta_1$ = 138 ppm, $\delta_2$ = 141 ppm. Die chemische Verschiebung wurde gegen $H_3PO_4$ extern bestimmt.

Beispiel 2

Propen wurde in Gegenwart einer Mischung aus Rh$(CO)_2$-acetylacetonat und dem Bisphosphitliganden der Formel (2) in 100 ml Texanol® in einem magnetisch gerührten 300 ml-Autoklaven hydroformyliert, wobei die Reaktionsbedingungen des Beispiels 1 entsprechend eingehalten wurden. Die Bestimmung der Reaktionsprodukte erfolgte gaschromatographisch.

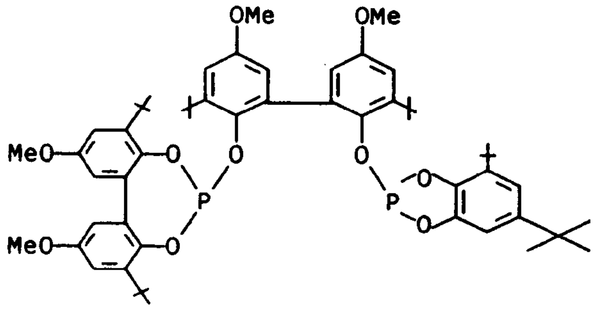

2

Man erhielt bei einem L/Rh-Verhältnis = 4,8 (mol/mol) eine Ausbeute an Butyraldehyd von 95 % mit einem n-Anteil von 66 %.

Der Bisphosphitligand (2) wurde auf folgende Weise synthetisiert: Aus einer Mischung von 35,8 g (0,1 mol) 2,2'-Dihydroxy-3,3'-di-tert.-butyl-5,5'-dimethoxy-1,1'-biphenyl mit 500 ml Toluol wurden 75 ml Toluol zur azeotropen Abtrennung von Wasser abdestilliert; die Lösung wurde mit 140 ml (1,0 mol) Triethylamin versetzt und bei -40°C zu einer Lösung von 13,8 g (0,1 mol) Phosphortrichlorid in 1 l Toluol innerhalb 30 min zugetropft. Nach langsamem Erwärmen und Rühren (1 h) bei Raumtemperatur erhitzte man 3 h zum Rückfluß. Zu dieser Mischung wurde bei -20°C innerhalb von 30 min eine Lösung aus 35,8 g (0,1 mol) 2,2'-Dihydroxy-3,3'-di-tert.-butyl-5,5'-dimethoxy-1,1'-biphenyl und 70 ml (0,5 mol) Triethylamin in 500 ml Toluol zugesetzt. Die Mischung wurde auf Raumtemperatur gebracht und 3 h am Rückfluß zum Sieden erhitzt. Bei -20°C erfolgte dann der Zusatz von 13,8 g (0,1 mol) Phosphortrichlorid in 100 ml Toluol innerhalb von 30 min. Die Mischung wurde auf Raumtemperatur gebracht und anschließend 1 h am Rückfluß erhitzt. Bei -20°C wurden innerhalb 30 min 22,2 g (0,1 mol) 1,2-Dihydroxy-3,5-di-tert.-butyl-benzol und 140 ml (1,0 mol) Triethylamin in 450 ml Toluol gesetzt. Nach dem Erwärmen auf Raumtemperatur wurde 3 h zum Rückfluß erhitzt.

Die Reaktionsmischung wurde heiß abfiltriert und das klare Filtrat unter vermindertem Druck vom Lösungsmittel befreit. Der Rückstand wurde in 500 ml heißem Acetonitril gelöst und heiß abfiltriert. In der Kälte kristallisierte (2) als weißer Feststoff aus (Schmelzpunkt: 162°C), der mit 60 % Ausbeute isoliert werden konnte.

$^{31}P$-NMR ($CDCl_3$): $\delta_1$ = 141 ppm, $\delta_2$ = 142 ppm. Die chemische Verschiebung wurde gegen $H_3PO_4$ extern bestimmt.

Beispiel 3

Propen wurde in Gegenwart einer Mischung aus $Rh(CO)_2$-acetylacetonat und dem Bisphosphitliganden der Formel (3) in 100 ml Texanol® in einem magnetisch gerührten 300 ml-Autoklaven hydroformyliert, wobei die Reaktionsbedingungen denen des Beispiels 1 entsprachen. Die Bestimmung der Reaktionsprodukte erfolgte gaschromatographisch.

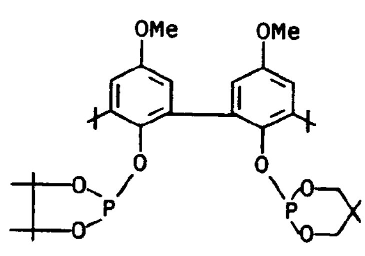

3

Man erhielt bei einem L/Rh-Verhältnis = 4,1 (mol/mol) eine Ausbeute an Butyraldehyd von 81 % mit einem n-Anteil von 91 %.

Der Bisphosphitligand wurde auf folgende Weise synthetisiert: 13 g (0,11 mol) 2,3-Dimethyl-2,3-butandiol wurden in 400 ml Toluol aufgenommen und 100 ml Toluol zur azeotropen Trocknung abdestilliert. Nach Zusatz von 154 ml (1,1 mol) Triethylamin wurde die Lösung zu 15,1 g (0,11 mol) Phosphortrichlorid gelöst in 1 l Toluol bei -40°C innerhalb von 30 min zugetropft. Anschließend wurde die Mischung auf Raumtemperatur erwärmt und 1,5 h zum Rückfluß erhitzt. Die Mischung wurde bei -40°C innerhalb 1 h mit 39,4 g (0,11 mol) 2,2'-Dihydroxy-3,3'-di-tert.-butyl-5,5'-dimethoxy-1,1'-biphenyl und 77 ml (0,55 mol) Triethylamin in 450 ml Toluol versetzt, auf Raumtemperatur gebracht und 3 h unter Rückfluß gerührt. Anschließend erfolgte innerhalb von 0,5 h der Zusatz von 15,1 g (0,11 mol) Phosphortrichlorid in 500 ml Toluol bei -40°C. Nach dem Erwärmen auf Raumtemperatur wurde die Mischung 4 h zum Rückfluß erhitzt. Nach Abkühlen auf -40°C ließ man 11,4 g (0,11 mol) 2,2-Dimethyl-1,3-propandiol und 154 ml (1,1 mol) Triethylamin in 400 ml Toluol zu der Reaktionsmischung zutropfen (0,5 h). Nach Erwärmen auf Raumtemperatur wurde 4 h zum Rückfluß erhitzt. Die Mischung wurde heiß filtriert und das klare Filtrat unter vermindertem Druck vom Lösungsmittel befreit. Der Rückstand wurde mit 250 ml Acetonitril extrahiert. Bei dem verbleibenden weißen Feststoff handelte es sich um (3), das mit 47 % Ausbeute isoliert werden konnte. (Schmelzpunkt: 158°C)

$^{31}$P-NMR-Daten ($CDCl_3$): $\delta_1$ = 118 ppm, $\delta_2$ = 142 ppm. Die chemische Verschiebung wurde gegen $H_3PO_4$ extern bestimmt.

Beispiel 4

Propen wurde in Gegenwart einer Mischung aus $Rh(CO)_2$-acetylacetonat und dem Bisphosphitliganden der Formel (4) in 100 ml Texanol® in einem magnetisch gerührten 300 ml-Autoklaven hydroformyliert, wobei die Reaktionsbedingungen denen des Beispiels 1 entsprachen. Die Bestimmung der Reaktionsprodukte erfolgte gaschromatographisch.

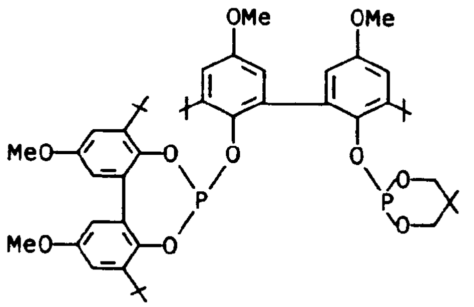

4

Man erhielt bei einem L/Rh-Verhältnis = 4,9 (mol/mol) eine Ausbeute an Butyraldehyd von 94 % mit einem n-Anteil von 68 %.

Der Bisphosphitligand (4) wurde auf folgende Weise synthetisiert: Eine Lösung von 71,6 g (0,2 mol) 2,2'-Dihydroxy-3,3'-di-tert.-butyl-5,5'-dimethoxy-1,1'-biphenyl in 600 ml Toluol wurde nach der Abdestillation von 75 ml Toluol zur azeotropen Trocknung mit 280 ml (2,0 mol) Triethylamin versetzt und bei -30°C innerhalb von 20 min zu einer Lösung von 27,5 g (0,2 mol) Phosphortrichlorid in 1 l Toluol zugetropft. Nach dem Erwärmen auf Raumtemperatur wurde 3 h zum Rückfluß erhitzt. Anschließend wurde die Mischung bei -20°C mit 71,6 g (0,2 mol) 2,2'-Dihydroxy-3,3'-di-tert.-butyl-5,5'-dimethoxy-1,1'-biphenyl und 140 ml (1,0 mol) Triethylamin in 400 ml Toluol innerhalb 20 min versetzt, auf Raumtemperatur erwärmt und 3 h zum Rückfluß erhitzt. Nach dem Abkühlen auf -25°C wurden 27,5 g (0,2 mol) Phosphortrichlorid in 100 ml Toluol innerhalb von 10 min zugetropft. Nach dem Erwärmen auf Raumtemperatur wurde 2 h zum Rückfluß erhitzt. Dann wurden 20,8 g (0,2 mol) 2,2-Dimethyl-1,3-propandiol und 280 ml (2,0 mol) Triethylamin in 400 ml Toluol bei -20°C innerhalb von 10 min zugetropft. Nach dem Erwärmen auf Raumtemperatur erhitzte man 2,5 h zum Rückfluß. Die Mischung wurde heiß filtriert und das klare Filtrat im Vakuum vom Lösungsmittel befreit. Der Rückstand wurde in 1 l heißem Acetonitril gelöst und filtriert; in der Kälte kristallisierte (4) als weißer Feststoff (Schmelzpunkt: 210°C) aus (Ausbeute: 70 %).

$^{31}$P-NMR-Daten ($CDCl_3$): $\delta_1$ = 120 ppm, $\delta_2$ = 142 ppm. Die chemische Verschiebung wurde gegen $H_3PO_4$ extern bestimmt.

Beispiel 5 und 6

Die Bisphosphitliganden 5 und 6 wurden wie in Beispiel 1 beschrieben bei der diskontinuierlichen Hydroformylierung von Propen eingesetzt.

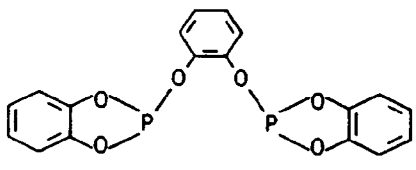

5

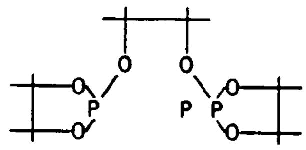

6

Die Reaktionsbedingungen und Ergebnisse sind Tabelle 1 zu entnehmen. Die Bestimmung der Reaktionsprodukte erfolgte gaschromatographisch.

Tabelle 1

| Ligand | L/Rh-Verh. (mol/mol) | Ausbeute BA (%) | n-Anteil (%) |
|---|---|---|---|
| 5 | 4,7 | 99 | 60 |
| 6 | 4,9 | 97 | 71 |

Reaktionsbedingungen: 90 ppm Rh, 100°C, 10 bar $CO:H_2$ (Molverhältnis 1:1), 45 bar Gesamtdruck, 4 h, 1,0 mol Propen, 100 ml Texanol®

Vergleichsbeispiel

Dieses Beispiel zeigt die katalytische Aktivität von zwei in EP-A-214622 als Hydroformylierungskokatalysatoren beschriebenen Bisphosphiten, die zu Vergleichszwecken synthetisiert und getestet wurden. Die Versuchsdurchführung erfolgte wie in Beispiel 2 beschrieben.

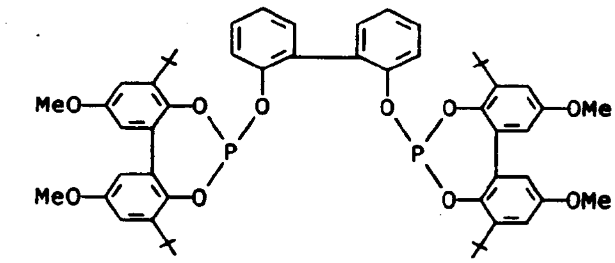

7

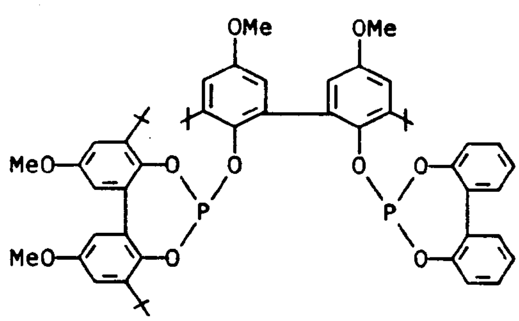

8

Die Reaktionsbedingungen und Ergebnisse sind Tabelle 2 zu entnehmen. Die Bestimmung der Reaktionsprodukte erfolgte gaschromatographisch.

Tabelle 2

| Ligand | L/Rh-Verh. (mol/mol) | Ausbeute BA (%) | n-Anteil (%) |
|---|---|---|---|
| 7 | 3,5 | 82 | 58 |
| 8 | 5,1 | 86 | 91 |

Reaktionsbedingungen: 90 ppm Rhodium, 70°C, 10 bar $CO:H_2$ (Molverhältnis 1 : 1), 30 bar Gesamtdruck, 4 h, 1,0 mol Propen, 100 ml Texanol®.

Beispiel 7

In einem Versuchsreaktor wurde zur kontinuierlichen Hydroformylierung von Propen dem Reaktor Kohlenmonoxid-Wasserstoff-Gasgemisch, Wasserstoff und Kreisgas in einer Menge zugeführt, daß bei einer konstanten Kreisgasmenge von 475 l/h und einer Abgasmenge von 30 l/h der Kohlenmonoxid-Gehalt im Kreisgas 4 bis 5 Vol.-% betrug. Der flüssige Reaktoraustrag wurde entspannt und das Aldehydprodukt in einem Fallfilmverdampfer abgetrennt. Der katalysatorhaltige Sumpf wurde kontinuierlich in den Reaktor zurückgeführt. Als Katalysator wurde das Bisphosphit (1) gemäß Beispiel 1 eingesetzt.

Die Ergebnisse und Reaktionsbedingungen sind Tabelle 3 zu entnehmen. Die Bestimmung der Reaktionsprodukte erfolgte gaschromatographisch.

Tabelle 3

| t (d) | Propen (g/h) | Umsatz (%) | BA-Ausb. (%) | n-Ant. (%) | RZA (g/l·h) | Propan Ausb. (%) | Rh (ppm) |
|---|---|---|---|---|---|---|---|
| 1 | 165 | 98 | 90 | 85 | 240 | 0,9 | 142 |
| 2 | 188 | 99 | 91 | 84 | 278 | 0,5 | |
| 3 | 184 | 99 | 94 | 83 | 281 | 0,6 | |
| 4 | 218 | 99 | 94 | 86 | 330 | 0,9 | |
| 5 | 215 | 99 | 93 | 87 | 323 | 1,2 | |
| 6 | 217 | 99 | 93 | 88 | 325 | 1,2 | |
| 7 | 214 | 99 | 93 | 88 | 321 | 1,5 | 127 |

Reaktionsbedingungen: Lösungmittel Texanol® und Butyraldehyd, 4,14 mmol Rh als $[Rh(OAc)_2]_2$, 36,5 g (41,4 mmol) Bisphosphit (1), L/Rh-Verh. = 10 : 1 (mol/mol), T = 60 - 70 °C, p = 20 bar, Kreisgas: 475 l/h, Abgas: 30 l/h, Katalysatorrücklauf: 600 ml/h.

Beispiel 8

Die Versuchsdurchführung entsprach der des Beispiels 7. Als Ligand wurde das Bisphosphit 2 eingesetzt. Der Kohlenmonoxid-Gehalt im Kreisgas betrug 13 bis 14 Vol.-%. Die Ergebnisse und Reaktionsbedingungen sind Tabelle 4 zu entnehmen. Die Bestimmung der Reaktionsprodukte erfolgte gaschromatographisch.

Tabelle 4

| t (d) | Propen (g/h) | Umsatz (%) | BA-Ausb. (%) | n-Ant. (%) | RZA (g/l·h) | Propan Ausb. (%) | Rh (ppm) |
|---|---|---|---|---|---|---|---|
| 1 | 235 | 98 | 92 | 72 | 349 | 2,6 | 131 |
| 2 | 235 | 98 | 94 | 74 | 355 | 1,7 | - |
| 3 | 236 | 99 | 96 | 72 | 367 | 0,8 | - |
| 4 | 233 | 98 | 95 | 73 | 358 | 1,5 | - |
| 5 | 232 | 98 | 94 | 74 | 353 | 2,2 | - |
| 6 | 234 | 98 | 92 | 75 | 346 | 2,5 | - |

Reaktionsbedingungen: Lösungsmittel Texanol® und Butyraldehyd, 4,14 mmol Rh als $[Rh(OAc)_2]_2$, 41,2 g (41,4 mmol) Bisphosphit (2), L/Rh-Verh. = 10:1 (mol/mol), T = 70 bis 80°C, p = 20 bar,
Kreisgas: 475 l/h, Abgas: 30 l/h, Katalysatorrücklauf: 600 ml/h.

Vergleichsbeispiel

Dieses Beispiel zeigt die katalytische Aktivität von dem in EP-A-214 622 beschriebenen Bisphosphit 8.

Die Versuchsdurchführung erfolgte entsprechend Beispiel 6. Der Kohlenmonoxid-Gehalt im Kreisgas betrug 4 bis 5 Vol.-%. Die Ergebnisse und Reaktionsbedingungen sind Tabelle 5 zu entnehmen. Die Bestimmung der Reaktionsprodukte erfolgte gaschromatographisch.

Tabelle 5

| t (d) | Propen (g/h) | Umsatz (%) | BA-Ausb. (%) | n-Ant. (%) | RZA (g/l·h) | Propan Ausb. (%) | Rh (ppm) |
|---|---|---|---|---|---|---|---|
| 1 | 230 | 93 | 83 | 96 | 310 | 2,7 | 130 |
| 2 | 229 | 94 | 84 | 96 | 310 | 2,7 | - |
| 3 | 228 | 94 | 83 | 96 | 304 | 2,9 | - |
| 4 | 231 | 94 | 81 | 96 | 304 | 2,9 | - |
| 5 | 234 | 93 | 82 | 96 | 308 | 2,8 | - |
| 6 | 231 | 94 | 82 | 96 | 307 | 2,7 | - |

Reaktionsbedingungen: Lösungsmittel Texanol® und Butyraldehyd, 4,14 mmol Rh als $[Rh(OAc)_2]_2$, 15,9 g (16,56 mmol) Bisphosphit (8),
L/Rh-Verh. = 4,0:1 (mol/mol), T = 90 bis 95°C, p = 20 bar.
Kreisgas: 475 l/h, Abgas: 30 l/h, Katalysatorrücklauf: 600 ml/h.

Beispiel 9

Die Bisphosphitliganden 1 bis 4 wurden wie in Beispiel 1 beschrieben bei der diskontinuierlichen Hydroformylierung von trans-2-Buten eingesetzt. Die Reaktionsbedingungen und Ergebnisse sind Tabelle 6 zu entnehmen. Die Bestimmung der Reaktionsprodukte erfolgte gaschromatographisch.

Tabelle 6

| Ligand | L/Rh-Verh. (mol/mol) | Ausbeute (Pentanal) (%) | n-Anteil (%) |
|---|---|---|---|
| 1 | 4,8 | 91 | 41 |
| 2 | 4,9 | 89 | 52 |
| 3 | 4,8 | 51 | 31 |
| 4 | 4,9 | 58 | 35 |

Reaktionsbedingungen: 90 ppm Rh, 90°C, 13 bar $CO:H_2$ (Molverhältnis 1:1), 25 bar Gesamtdruck, 4 h, 1,0 mol trans-2-Buten; 90 ml Texanol®.

Vergleichsbeispiel

Dieses Beispiel zeigt die katalytische Aktivität von dem in EP-A-214 622 als Hydroformylierungskatalysator beschriebenen Bisphosphit 8, das zu Vergleichszwecken synthetisiert und getestet wurde.

Die Reaktionsbedingungen und Ergebnisse sind Tabelle 7 zu entnehmen. Die Bestimmung der Reaktionsprodukte erfolgte gaschromatographisch.

Tabelle 7

| Ligand | L/Rh-Verh. (mol/mol) | Ausbeute (Pentanal) (%) | n-Anteil (%) |
|---|---|---|---|
| 8 | 4,7 | 31 | 37 |

Reaktionsbedingungen: 90 ppm Rh, 90°C, 13 bar $CO:H_2$ (Molverhältnis 1:1), 25 bar Gesamtdruck, 4 h, 1,0 mol trans-2-Buten; 90 ml Texanol®.

**Patentansprüche**

1. Rhodiumhydroformylierungskatalysator mit Bis-phosphit-liganden der Formel I

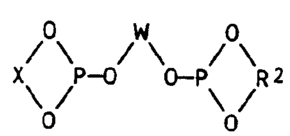

I,

in der

| | |
|---|---|
| -X- | einen zweiwertigen Bisarylenrest oder $R^1$, |
| W | einen zweiwertigen Arylen-, Bisarylen- oder Alkylen-Rest und |
| $R^1$ und $R^2$ | gleich oder verschieden sind und einen substituierten oder unsubstituierten Alkylen oder ortho-Arylenrest bedeuten. |

2. Rhodiumhydroformylierungskatalysator mit Bis-phosphit-liganden gemäß Anspruch 1 der Formel I, dadurch gekennzeichnet, daß X und W Bis-arylenreste bedeuten.

3. Rhodiumhydroformylierungskatalysator mit Bis-phosphit-liganden gemäß Anspruch 1 der Formel I, dadurch gekennzeichnet, daß X und W Reste der Formel

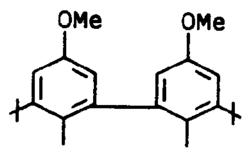

und

$R^2$ einen o-Phenylen-, 2,2-Dimethyl-propylen-1,3- oder 1,1,2,2-Tetramethyl-ethylen-Rest bedeuten.

4. Rhodiumhydroformylierungskatalysator mit Bis-phosphit-liganden gemäß Anspruch 1 der Formel I, in der X, W und $R^2$ unabhängig voneinander o-Phenylen, 2,2-Dimethyl-propylen-1,3- oder 1,1,2,2-Tetramethyl-ethylen bedeuten.

5. Rhodiumhydroformylierungskatalysator mit Bis-phosphit-liganden gemäß Anspruch 1 der Formel 1, dadurch gekennzeichnet, daß W den Rest der Formel

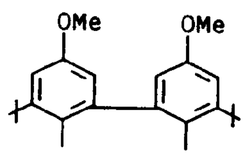

und X und $R^2$ unabhängig voneinander einen o-Phenylen-, 2,2-Dimethyl-propylen-1,3- oder 1,1,2,2-Tetramethyl-ethylenrest bedeuten.

6. Rhodiumhydroformylierungskatalysator mit Bis-phosphit-liganden gemäß Anspruch 5, dadurch gekennzeichnet, daß W den o-Phenylen-, 2, 2-Dimethyl-propylen-1,3- oder 1,1,2,2-Tetramethyl-ethylenrest bedeutet.

7. Verfahren zur Hydroformylierung von Olefinen mit 2 bis 20 C-Atomen, dadurch gekennzeichnet, daß man die Umsetzung in Gegenwart eines Hydroformylierungskatalysators gemäß Anspruch 1 durchführt.

8. Verbindung der Formel (1)

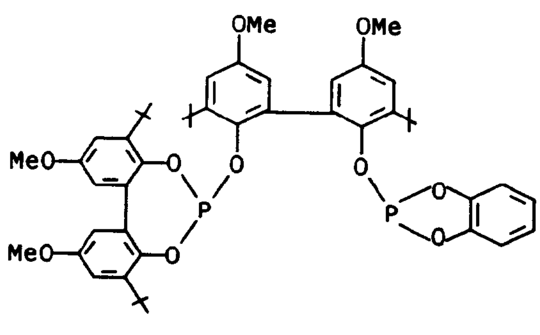

1

9. Verbindung der Formel (2)

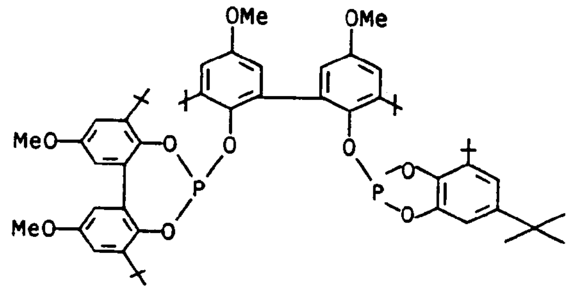

2

**10.** Verbindung der Formel (3)

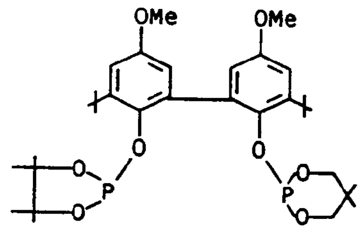

3

**11.** Verbindung der Formel (4)

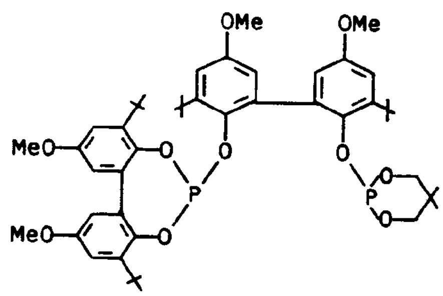

4

## Claims

**1.** A rhodium hydroformylation catalyst containing bisphosphite ligands of the formula I

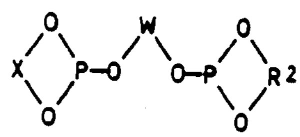

I,

where

| | |
|---|---|
| -X- | is a divalent bisarylene radical or $R^1$, |
| W | is a divalent arylene, bisarylene or alkylene radical, and |
| $R^1$ and $R^2$ | are identical or different and are substituted or unsubstituted alkylene or ortho-arylene. |

**2.** A rhodium hydroformylation catalyst containing bisphosphite ligands as claimed in claim 1 of the formula I, wherein X and W are bisarylene.

**3.** A rhodium hydroformylation catalyst containing bisphosphite ligands as claimed in claim 1 of the formula I, wherein X and W are radicals of the formula

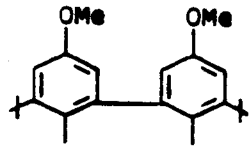

and

$R^2$ is o-phenylene, 2,2-dimethyl-1,3-propylene or 1,1,2,2-tetramethylethylene.

4. A rhodium hydroformylation catalyst containing bisphosphite ligands as claimed in claim 1 of the formula I, where X, W and $R^2$, independently of one another, are o-phenylene, 2,2-dimethyl-1,3-propylene or 1,1,2,2-tetramethylethylene.

5. A rhodium hydroformylation catalyst containing bisphosphite ligands as claimed in claim 1 of the formula I, wherein W is the radical of the formula

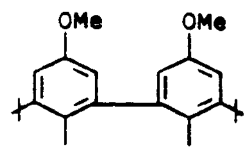

and X and $R^2$, independently of one another, are o-phenylene, 2,2-dimethyl-1,3-propylene or 1,1,2,2-tetramethylethylene.

6. A rhodium hydroformylation catalyst containing bisphosphite ligands as claimed in claim 5, wherein W is o-phenylene, 2,2-dimethyl-1,3-propylene or 1,1,2,2-tetramethylethylene.

7. A process for hydroformylating olefins having from 2 to 20 carbon atoms, which comprises carrying out the reaction in the presence of a hydroformylation catalyst as claimed in claim 1.

8. The compound of the formula (1)

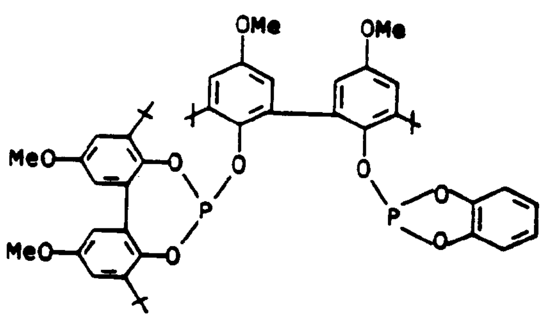

1

9. The compound of the formula (2)

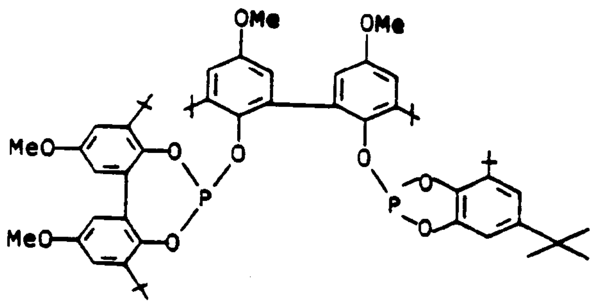

2

**10.** The compound of the formula (3)

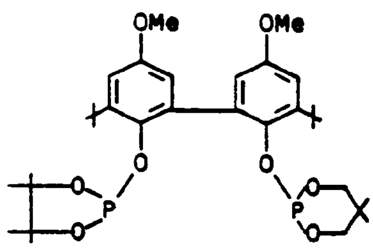

3

**11.** The compound of the formula (4)

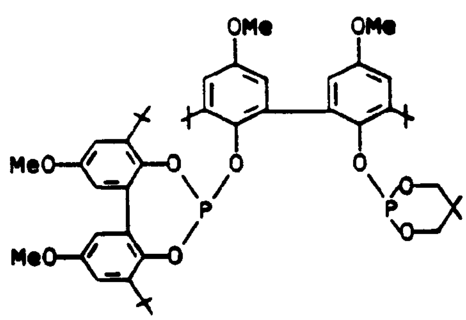

4

## Revendications

**1.** Catalyseur d'hydroformylation au rhodium à ligands de bis-phosphite de formule I

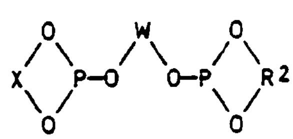

I,

dans laquelle

| | |
|---|---|
| -X- | est un reste bisarylène bivalent ou $R^1$, |
| W | est un reste arylène, bisarylène ou alkylène bivalent et |
| $R^1$ et $R^2$ | sont identiques ou différents et représentent chacun un reste alkylène ou ortho-arylène substitué ou non substitué. |

2. Catalyseur d'hydroformylation au rhodium à ligands de bis-phosphite selon la revendication 1 de formule I, caractérisé en ce que X et W représentent des restes bisarylène.

3. Catalyseur d'hydroformylation au rhodium à ligands de bis-phosphite selon la revendication 1 de formule I, caractérisé en ce que X et W représentent des restes de formule

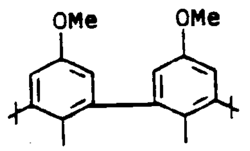

et $R^2$ représente un reste o-phénylène, 2,2-diméthylpropylène-1,3 ou 1,1,2,2-tétraméthyléthylène.

4. Catalyseur d'hydroformylation au rhodium à ligands de bis-phosphite selon !a revendication 1 de formule I, dans laquelle X, W et $R^2$ représentent chacun, indépendamment les uns des autres, un reste o-phénylène, 2,2-diméthylpropylène-1,3 ou 1,1,2,2-tétraméthyléthylène.

5. Catalyseur d'hydroformylation au rhodium à ligands de bis-phosphite selon la revendication 1 de formule I, caractérisé en ce que W représente le reste de formule

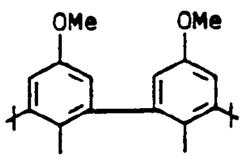

et X et $R^2$ représentent chacun, indépendamment l'un de l'autre, un reste o-phénylène, 2,2-diméthylpropylène-1,3 ou 1,1,2,2-tétraméthyléthylène.

6. Catalyseur d'hydroformylation au rhodium à ligands de bis-phosphite selon la revendication 5, caractérisé en ce que W représente le reste o-phénylène, 2,2-diméthylpropylène-1,3 ou 1,1,2,2-tétraméthyléthylène.

7. Procédé d'hydroformylation d'oléfines à 2-20 atomes de carbone, caractérisé en ce qu'on conduit la réaction en présence d'un catalyseur d'hydroformylation selon la revendication 1.

8. Composé de formule (1)

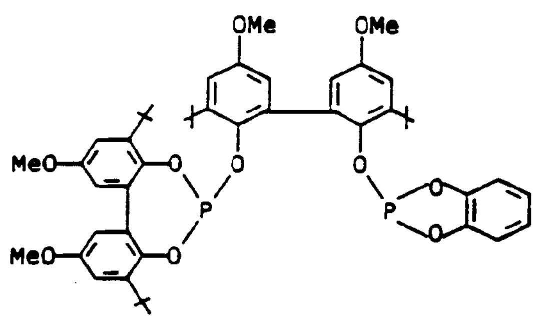

1

9. Composé de formule (2)

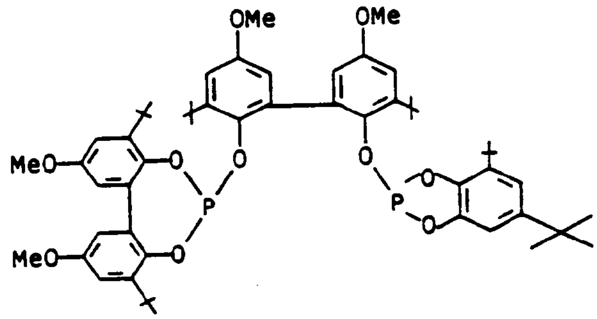

2

**10.** Composé de formule (3)

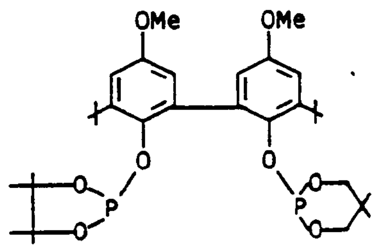

3

**11.** Composé de formule (4)

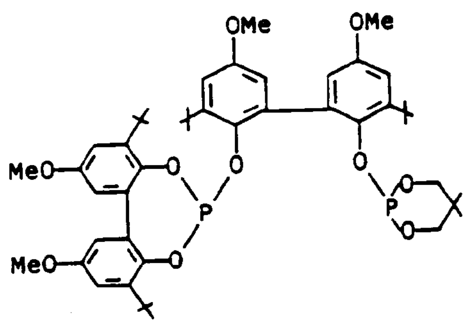

4